# EUROPEAN PATENT APPLICATION

(11) **EP 3 543 361 A1**
(43) Date of publication of application: **25.09.2019**
(21) Application number: 19162037.6
(22) Date of filing: 14.10.2015
(51) Int. Cl.: C12Q 1/6886

(54) **METHODS AND COMPOSITIONS FOR CORRELATING GENETIC MARKERS WITH COLORECTAL CANCER RISK**

(30) Priority: 14.10.2014 US 201462063745 P
(62) Divisional of application: 15851169.1
(71) Applicant: Wake Forest University Health Sciences, Winston-Salem, NC 27157 (US)
(72) Inventor: XU, Jianfeng, Winston-Salem, North Carolina 27157 (US); SUN, Jielin, Winston-Salem, North Carolina 27157 (US); ZHENG, Siqun Lilly, Winston-Salem, North Carolina 27157 (US); CHEN, Zhuo, Winston-Salem, North Carolina 27157 (US); WANG, Li, Winston-Salem, North Carolina 27157 (US); ZHANG, Zheng, Winston-Salem, North Carolina 27157 (US)
(74) Representative: HGF Limited

(57) **Abstract**

The present invention provides methods of assessing an individual subject's risk of developing different types of cancer, comprising calculating a genetic risk score (GRS) for the subject.

## Description

### Statement of Priority

This application claims the benefit, under 35 U.S.C. § 119(e), of U.S. Provisional Application Serial No. 62/063,745, filed October 14, 2014, the entire contents of which are incorporated by reference herein.

### Field of the Invention

The present invention provides methods and compositions directed to assessing risk of having or developing various types of cancer by analyzing multiple single nucleotide polymorphisms (SNPs) in nucleic acid of a subject.

### Background of the Invention

Single nucleotide polymorphisms (SNPs) are stable genetic markers throughout the human genome, which can be tested for their association with various disease traits. These markers can be tested at birth and will not change in a patient's lifetime and thus represent a new form of biomarkers that predict lifetime risk to disease as opposed to an immediate risk.

Numerous cancer risk-associated SNPs have been discovered from genome-wide association studies (GWAS). Although each of these SNPs is only moderately associated with risk of a particular cancer, a genetic risk score (GRS) based on a combination of risk-associated SNPs can be used to identify an individual's risk for a variety of different cancers. These risk-associated SNPs have broad practical applications because they are common in the general population.

The present invention overcomes previous shortcomings in the art by identifying significant statistical associations between multiple genetic markers and cancer risk for a variety of different cancers.

### Summary of the Invention

In one embodiment, the present invention provides a method of producing a personalized cancer risk report for a subject, comprising: a) determining, from a nucleic acid sample obtained from the subject, a genotype for the subject at a plurality of biallelic polymorphic loci, wherein each locus of said plurality has an associated allele and an unassociated allele, wherein the genotype is selected from the group consisting of homozygous for the associated allele, heterozygous, and homozygous for the unassociated allele and wherein said plurality of biallelic polymorphic loci is a multiplicity, in any combination, of the single nucleotide polymorphisms in each of Table 1 (breast), Table 2 (lung), Table 3 (colorectal), Table 4 (prostate), Table 5 (glioma), Table 6 (neuroblastoma), Table 7 (chronic lymphocytic leukemia), Table 8 (pancreatic), Table 9 (non-Hodgkin lymphoma), Table 10 (bladder), Table 11 (renal) Table 12 (ovarian), Table 13 (melanoma), Table 14 (Hodgkin lymphoma), Table 15 (acute lymphocytic leukemia), Table 16 (thyroid), and Table 17 (testicular); b) calculating a genetic risk score (GRS) for the subject based on the genotype determined for each plurality of biallelic polymorphic loci of step (a); and c) producing a personalized cancer risk report for the subject based on the GRS calculated in step (b).

In another embodiment, the present invention provides a method of identifying a subject as having an increased risk of developing breast cancer, lung cancer, colorectal cancer, prostate cancer, glioma, neuroblastoma, chronic lymphocytic leukemia, pancreatic cancer, non-Hodgkin lymphoma, bladder cancer, renal cancer, ovarian cancer, melanoma, Hodgkin lymphoma, acute lymphocytic leukemia, thyroid cancer and/or testicular cancer, comprising: a) determining, from a nucleic acid sample obtained from the subject, a genotype for the subject at a plurality of biallelic polymorphic loci, wherein each locus of said plurality has an associated allele and an unassociated allele, wherein the genotype is selected from the group consisting of homozygous for the associated allele, heterozygous, and homozygous for the unassociated allele and wherein said plurality of biallelic polymorphic loci is a multiplicity, in any combination, of the single nucleotide polymorphisms in each of Table 1 (breast), Table 2 (lung), Table 3 (colorectal), Table 4 (prostate), Table 5 (glioma), Table 6 (neuroblastoma), Table 7 (chronic lymphocytic leukemia), Table 8 (pancreatic), Table 9 (non-Hodgkin lymphoma), Table 10 (bladder), Table 11 (renal) Table 12 (ovarian), Table 13 (melanoma), Table 14 (Hodgkin lymphoma), Table 15 (acute lymphocytic leukemia), Table 16 (thyroid) and/or Table 17 (testicular); and b) calculating a genetic risk score (GRS) for the subject based on the genotype determined for each plurality of biallelic polymorphic loci in step (a), wherein a GRS of greater than 1.0 identifies the subject as having an increased risk of developing the type of cancer associated with said GRS of greater than 1.0, thereby identifying the subject as having an increased risk of developing breast cancer, lung cancer, colorectal cancer, prostate cancer, glioma, neuroblastoma, chronic lymphocytic leukemia, pancreatic cancer, non-Hodgkin lymphoma, bladder cancer, renal cancer, ovarian cancer, melanoma, Hodgkin lymphoma, acute lymphocytic leukemia, thyroid cancer and/or testicular cancer.

In further embodiments, the present invention provides a kit comprising reagents and instructions for carrying out the methods of this invention.

The present invention also provides a computer program product comprising: a computer readable storage medium having computer readable code embodied in the medium, the computer code comprising: computer readable code to perform operations to carry out the methods of this invention.

Further provided herein is a computer system, comprising: a processor; and a memory coupled to the processor, the memory comprising computer readable program code embodied therein that, when executed by the processor, causes the processor to perform operations to carry out the methods of this invention.

### Brief Description of the Drawings

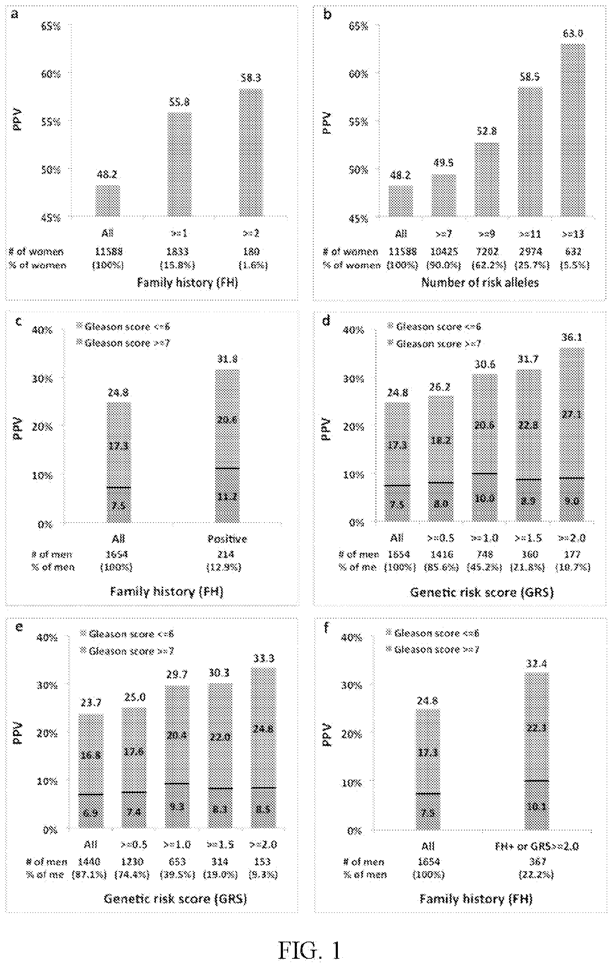

Fig. 1. Positive predictive values (PPV) of family history and risk-associated SNPs for a diagnosis of breast cancer (a-b) and prostate cancer (c-f) from a re-analysis of two published studies.³⁻⁴ PPV are presented based on a) family history of breast cancer, b) number of 10 breast cancer risk-associated alleles, c) family history of prostate cancer, d) genetic risk score (GRS) calculated from 33 prostate cancer risk-associated SNPs in the entire cohort, e) GRS among subjects with a negative family history, and f) family history or GRS. The dark grey bars indicate PPV for Gleason score 7 or higher prostate cancer.
Fig. 2. Genetic risk score (GRS) performs better than family history (FH) in determining a subject's cancer risk. AUC = area under the curve.
Fig. 3. A non-limiting example of a Personalized Cancer Risk Report of this invention.

### Detailed Description of the Invention

The present invention is explained in greater detail below. This description is not intended to be a detailed catalog of all the different ways in which the invention may be implemented, or all the features that may be added to the instant invention. For example, features illustrated with respect to one embodiment may be incorporated into other embodiments, and features illustrated with respect to a particular embodiment may be deleted from that embodiment. In addition, numerous variations and additions to the various embodiments suggested herein will be apparent to those skilled in the art in light of the instant disclosure, which do not depart from the instant invention. Hence, the following specification is intended to illustrate some particular embodiments of the invention, and not to exhaustively specify all permutations, combinations and variations thereof.

The present invention is based on the unexpected discovery of a method of producing a personalized cancer risk report for a subject, comprising: a) determining, from a nucleic acid sample obtained from the subject, a genotype for the subject at a plurality of biallelic polymorphic loci, wherein each locus of said plurality has an associated allele and an unassociated allele, wherein the genotype is selected from the group consisting of homozygous for the associated allele, heterozygous, and homozygous for the unassociated allele and wherein said plurality of biallelic polymorphic loci is a multiplicity of the single nucleotide polymorphisms in each of Table 1 (breast), Table 2 (lung), Table 3 (colorectal), Table 4 (prostate), Table 5 (glioma), Table 6 (neuroblastoma), Table 7 (chronic lymphocytic leukemia), Table 8 (pancreatic), Table 9 (non-Hodgkin lymphoma), Table 10 (bladder), Table 11 (renal) Table 12 (ovarian), Table 13 (melanoma), Table 14 (Hodgkin lymphoma), Table 15 (acute lymphocytic leukemia), Table 16 (thyroid), and/or Table 17 (testicular), in any combination; and b) calculating a genetic risk score (GRS) for the subject based on the genotype determined for each plurality of biallelic polymorphic loci in step (a), thereby producing a personalized cancer risk report for the subject.

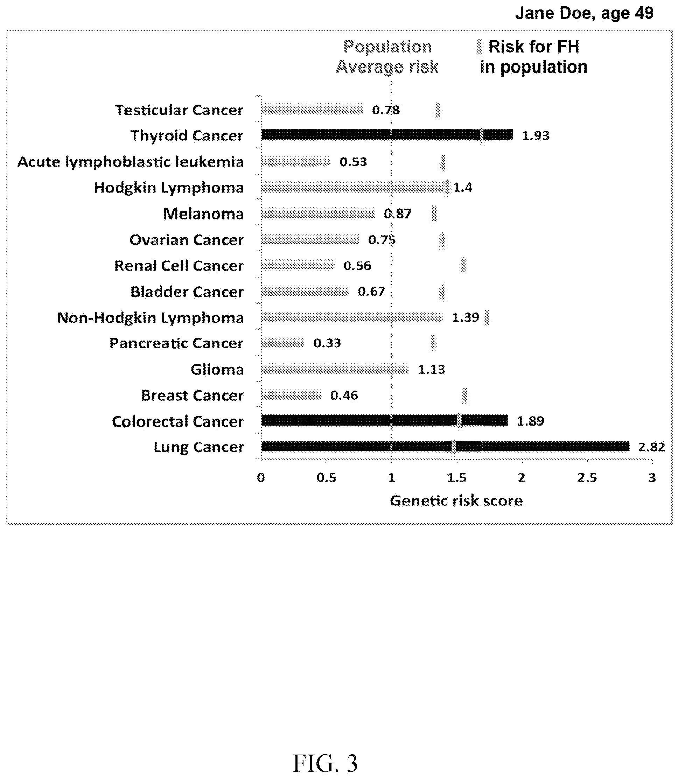

The present invention also provides a personalized cancer risk report that is produced by carrying out the methods described herein, comprising, consisting essentially of or consisting of: a) a first region comprising a listing of one or more of the following cancer types: breast cancer, lung cancer, colorectal cancer, prostate cancer, glioma, neuroblastoma, chronic lymphocytic leukemia, pancreatic cancer, non-Hodgkin lymphoma, bladder cancer, renal cancer, ovarian cancer, melanoma, Hodgkin lymphoma, acute lymphocytic leukemia, thyroid cancer and testicular cancer, in any combination and/or order; and b) a second region, adjacent to said first region, comprising a genetic risk score (GRS) value for each cancer type listed in said first region as calculated for a subject. In some embodiments the personalized cancer report can be in a graph format, with the first region and second region positioned as x and y axes relative to one another, in either orientation of the first region being on the x axis or the y axis and the second region being on the x axis or the y axis. In some embodiments, the personalized cancer risk report can comprise a mark identifying the value for the risk based on family history of each cancer type in a population. In some embodiments, the personalized cancer risk report can comprise a line (e.g., a solid line, dashed line, etc.) positioned above the genetic risk score value of 1.0, indicating the population average risk. In some embodiments, the personalized cancer risk report includes only the cancer types for which the subject has a calculated GRS of greater than 1.0. A nonlimiting example of a Personalized Cancer Risk Report of this invention is provided in Fig. 3.

In a further embodiment, the present invention provides a method of identifying a subject as having an increased risk of developing breast cancer, lung cancer, colorectal cancer, prostate cancer, glioma, neuroblastoma, chronic lymphocytic leukemia, pancreatic cancer, non-Hodgkin lymphoma, bladder cancer, renal cancer, ovarian cancer, melanoma, Hodgkin lymphoma, acute lymphocytic leukemia, thyroid cancer and/or testicular cancer, comprising: a) determining, from a nucleic acid sample obtained from the subject, a genotype for the subject at a plurality of biallelic polymorphic loci, wherein each locus of said plurality has an associated allele and an unassociated allele, wherein the genotype is selected from the group consisting of homozygous for the associated allele, heterozygous, and homozygous for the unassociated allele and wherein said plurality of biallelic polymorphic loci is a multiplicity of the single nucleotide polymorphisms in each of Table 1 (breast), Table 2 (lung), Table 3 (colorectal), Table 4 (prostate), Table 5 (glioma), Table 6 (neuroblastoma), Table 7 (chronic lymphocytic leukemia), Table 8 (pancreatic), Table 9 (non-Hodgkin lymphoma), Table 10 (bladder), Table 11 (renal) Table 12 (ovarian), Table 13 (melanoma), Table 14 (Hodgkin lymphoma), Table 15 (acute lymphocytic leukemia), Table 16 (thyroid) and/or Table 17 (testicular), in any combination; and b) calculating a genetic risk score (GRS) for the subject based on the genotype determined for each plurality of biallelic polymorphic loci in step (a), wherein a GRS of greater than 1.0 identifies the subject as having an increased risk of developing the type of cancer associated with said GRS of greater than 1.0, thereby identifying the subject as having an increased risk of developing breast cancer, lung cancer, colorectal cancer, prostate cancer, glioma, neuroblastoma, chronic lymphocytic leukemia, pancreatic cancer, non-Hodgkin lymphoma, bladder cancer, renal cancer, ovarian cancer, melanoma, Hodgkin lymphoma, acute lymphocytic leukemia, thyroid cancer and/or testicular cancer.

The present invention further provides a method of identifying a subject as a candidate for a clinical trial (e.g., for a cancer treatment, for a prophylactic cancer treatment, for a cancer vaccine, etc.), comprising: a) determining, from a nucleic acid sample obtained from the subject, a genotype for the subject at a plurality of biallelic polymorphic loci, wherein each of said plurality has an associated allele and an unassociated allele, wherein the genotype is selected from the group consisting of homozygous for the associated allele, heterozygous, and homozygous for the unassociated allele and wherein said plurality of biallelic polymorphic loci is a multiplicity of the single nucleotide polymorphisms in each of Table 1 (breast), Table 2 (lung), Table 3 (colorectal), Table 4 (prostate), Table 5 (glioma), Table 6 (neuroblastoma), Table 7 (chronic lymphocytic leukemia), Table 8 (pancreatic), Table 9 (non-Hodgkin lymphoma), Table 10 (bladder), Table 11 (renal) Table 12 (ovarian), Table 13 (melanoma), Table 14 (Hodgkin lymphoma), Table 15 (acute lymphocytic leukemia), Table 16 (thyroid) and/or Table 17 (testicular), in any combination; b) calculating a genetic risk score (GRS) for the subject based on the genotype determined for each plurality of biallelic polymorphic loci in step (a), wherein a GRS of greater than 1.0 identifies the subject as having an increased risk of developing the type of cancer associated with said GRS of greater than 1.0, thereby identifying the subject as having an increased risk of developing breast cancer, lung cancer, colorectal cancer, prostate cancer, glioma, neuroblastoma, chronic lymphocytic leukemia, pancreatic cancer, non-Hodgkin lymphoma, bladder cancer, renal cancer, ovarian cancer, melanoma, Hodgkin lymphoma, acute lymphocytic leukemia, thyroid cancer and/or testicular cancer; c) further evaluating the subject for the clinical trial and/or including the subject in the clinical trial, if the subject is identified as having an increased risk of developing the type of cancer associated with said GRS of greater than 1.0; and d) not further evaluating the subject for the clinical trial and/or not including the subject in the clinical trial if the subject is not identified as having an increased risk of developing breast cancer, lung cancer, colorectal cancer, prostate cancer, glioma, neuroblastoma, chronic lymphocytic leukemia, pancreatic cancer, non-Hodgkin lymphoma, bladder cancer, renal cancer, ovarian cancer, melanoma, Hodgkin lymphoma, acute lymphocytic leukemia, thyroid cancer and/or testicular cancer by the calculating step of (b). An example for inclusion would be to identify subjects at increased risk for whom prophylactic interventions could be developed. An exclusion criterion could be to exclude subjects at increased risk from clinical trials where development of specific oncologic disease would be detrimental to the study.

In the methods described above, in some embodiments, the determining step can comprise receiving genotype data from a genotyping apparatus, as would be well known in the art. Nonlimiting examples of genotyping protocols include, but are not limited to, restriction fragment length polymorphism identification (RFLPI) of genomic DNA, random amplified polymorphic detection (RAPD) of genomic DNA, amplified fragment length polymorphism detection (AFLPD), polymerase chain reaction (PCR), DNA sequencing, allele specific oligonucleotide (ASO) probes, and hybridization to DNA microarrays or beads. Accordingly genotyping apparatus would comprise any instruments, machines and/or devices employed in such genotyping protocols, as would be well known in the art.

In some embodiments of the method described herein, the plurality of biallelic polymorphic loci can include every single nucleotide polymorphism of Tables 1 through 17. In some embodiments, the plurality of biallelic polymorphic loci can exclude any of the single nucleotide polymorphisms of Tables 1 through 17, in any combination. For example, the plurality can be any number of different SNPs (at least, 2, 3, 4, etc.) from any number of different tables (at least, 2, 3, 4, 5, 6, 7, etc.) as provided herein, representing any combination of different cancers as provided herein. In some embodiments, the method can include an assessment of an individual's genotype at any SNP site in linkage disequilibrium (LD) with any of the SNPs in Tables 1 through 17.

In some embodiments of this invention, the subject is considered or identified to be at increased risk of having or developing lung cancer, colorectal cancer, prostate cancer, glioma, neuroblastoma, chronic lymphocytic leukemia, pancreatic cancer, non-Hodgkin lymphoma, bladder cancer, renal cancer, ovarian cancer, melanoma, Hodgkin lymphoma, acute lymphocytic leukemia, thyroid cancer and/or testicular cancer.

In some embodiments, the subject has a family history of breast cancer, lung cancer, colorectal cancer, prostate cancer, glioma, neuroblastoma, chronic lymphocytic leukemia, pancreatic cancer, non-Hodgkin lymphoma, bladder cancer, renal cancer, ovarian cancer, melanoma, Hodgkin lymphoma, acute lymphocytic leukemia, thyroid cancer and/or testicular cancer.

In some embodiments of this invention, the subject is not considered or identified to be at increased risk of having or developing breast cancer, lung cancer, colorectal cancer, prostate cancer, glioma, neuroblastoma, chronic lymphocytic leukemia, pancreatic cancer, non-Hodgkin lymphoma, bladder cancer, renal cancer, ovarian cancer, melanoma, Hodgkin lymphoma, acute lymphocytic leukemia, thyroid cancer and/or testicular cancer.

In some embodiments, the subject does not have a known family history of breast cancer, lung cancer, colorectal cancer, prostate cancer, glioma, neuroblastoma, chronic lymphocytic leukemia, pancreatic cancer, non-Hodgkin lymphoma, bladder cancer, renal cancer, ovarian cancer, melanoma, Hodgkin lymphoma, acute lymphocytic leukemia, thyroid cancer and/or testicular cancer.

The step of determining or detecting includes manipulating a fluid or tissue sample obtained from the subject to extract nucleic acid of the subject from the sample in a form that allows for the nucleotide sequence of the nucleic acid to be identified.

The genetic risk score (GRS) calculation is described in some embodiments as follows: a weighted genetic score is calculated for each subject based on the genotypes at the cancer risk-associated SNPs recited herein in Tables 1 through 17 and weighted by the respective odds ratio (OR) of each of these SNPs derived from an external study using a method described by Pharoah et al ("Polygenes, risk prediction, and targeted prevention of breast cancer" N Engl J Med 358:2796-2803 (2008)). Briefly, 1) the allelic OR for each SNP was obtained from an external study, 2) the genotypic OR of each SNP was estimated from the allelic OR assuming a multiplicative model, 3) the risk relative to the average risk in the population was calculated for each genotype based on genotypic OR and genotype frequency in the study population, and 4) genetic risk score was obtained by multiplying the risks relative to the population of all SNPs. Therefore, a genetic risk score of 1.0 indicates an average risk in the general population.

Furthermore, the heterozygous risk is the OR because the OR is the measure of association between a single risk allele (heterozygous genotype) and the outcome. The homozygous risk is when one has two risk alleles (homozygous genotype), which is the OR* OR or (OR²). A nonlimiting example of how the GRS of this invention is calculated is provided in the Examples section herein.

One objective of carrying out the methods of this invention is to identify a subject who is not otherwise identified or who may not otherwise be identified as being at increased risk or at high risk of having or developing breast cancer, lung cancer, colorectal cancer, prostate cancer, glioma, neuroblastoma, chronic lymphocytic leukemia, pancreatic cancer, non-Hodgkin lymphoma, bladder cancer, renal cancer, ovarian cancer, melanoma, Hodgkin lymphoma, acute lymphocytic leukemia, thyroid cancer and/or testicular cancer as a subject for whom screening protocols and preventive therapies and/or treatments would be beneficial. For example, using screening and treatment protocols that are otherwise used for high risk subjects on a subject identified according to the methods of this invention as having an increased risk of having or developing a particular type of cancer, but who would not otherwise be considered for screening and treatment protocols used for high risk subjects, has the benefit of allowing earlier detection and possibly even prevention of cancer in that subject. Under current protocols of standard care, such a subject would not be screened or treated in the same manner that a subject known to be a high risk subject would be screened or treated and therefore such a subject could develop cancer that could have been prevented and/or such a subject may have cancer detected at a later stage than may have been possible otherwise, with the outcome that treatment at such a later stage may be more complex, less successful and/or less likely to improve the subject's outcome. Thus, the present invention fulfills a long felt but unmet need of identifying subjects for whom screening and preventive treatment would be beneficial but for whom such screening and preventive treatment is not currently considered or made available because the subject does not otherwise qualify as a high risk subject. Such identification of these previously unrecognized subjects as having an increased risk of having or developing a particular cancer type based on the subject' GRS as calculated according to this invention has the added benefit of reducing mortality caused by the various cancers listed herein.

A further objective in carrying out the methods of the present invention is to reduce or minimize overscreening of subjects. For example, under current standard of care (SOC) guidelines, subjects are being over-screened/over-treated based, e.g., on family history (FH), causing undue cost and worry, and potentially resulting in dramatic interventional medical decisions (e.g. double mastectomy/bilateral oophorectomy). This is especially profound in light of recent data that shows that double mastectomies do not improve breast cancer survival (www.latimes.com/science/sciencenow/la-sci-sn-breast-cancer-double-mastectomy-20140903-story.html). Thus, use of a GRS as described herein provides a direct, individualized measurement of risk, and can therefore distinguish risk among, e.g., siblings. GRS measurement of risk also extends to non-related individuals, proving to be more effective as a measurement of individualized risk than family history. In particular, family history is limited to only a certain percentage of the population whereas GRS is universal and can be utilized with all individuals as a truly objective measurement.

Thus, the present invention further provides, in the methods described herein, the step of screening the subject for the cancer(s) associated with said GRS of greater than 1.0 according to a protocol recommended for a subject considered or identified to be at high risk or increased risk of having or developing the cancer(s) associated with said GRS of greater than 1.0.

Also provided herein, in the methods described herein, is the step of administering a prophylactic treatment, such as chemopreventive therapy, to the subject, wherein the treatment is specific for the cancer(s) associated with said GRS of greater than 1.0. Nonlimiting examples of primary chemoprevention include Tamoxifen, an oral selective antiestrogen agent (SERM) for estrogen receptor positive (ER+) breast cancer and the first chemoprevention drug to receive FDA approval; Raloxifene, another SERM which helps prevent breast cancer in postmenopausal women; 5-reductase inhibitors such as Finasteride, statin drugs, and natural compounds such as lycopene for prostate cancer; and nonsteroidal anti-inflammatory drugs (NSAIDs), such as celecoxib as one example, for colorectal cancer. Other chemopreventive therapy now known or later developed is included in the scope of this invention.

If a subject is identified as having an increased risk of having or developing a type of cancer as set forth in the methods of this invention, U.S. Preventive Services Task Force (USPSTF) screening guidelines can be applied, based on the recommendations for screening higher risk individuals. A subject of this invention can be screened in accordance with these guidelines and recommendations or a subject of this invention can be screened more frequently and/or more extensively than what these guidelines provide.

For acute lymphoblastic leukemia (ALL), there are no special tests recommended to detect acute lymphocytic leukemia (ALL) early. The best way to find leukemia early is to report any possible signs or symptoms of leukemia to the doctor right away.

For bladder cancer, screening of a subject at increased risk can include annual urinalysis, including microscopic examination for microhematuria and cytologic examination for neoplastic cells. Four intravesical drugs are available for chemotherapy: thiotepa, doxorubicin, mitomycin C, epirubicin, but studies have failed to show that these therapies reduce progression. European Organization for Research and Treatment of Cancer/Medical Research Council (EORTC/MRC) randomized clinical trials showed a long-term reduction in tumor recurrence of 6%. These drugs can be used as prophylactic treatment in high-risk patients after TUR. Alternating mitomycin C and Bacillus Calmette-Guerin (BCG) instillation prophylaxis treatments can be used for superficial bladder cancer.

For Hodgkin lymphoma, careful, regular medical checkups may be helpful for people at increased risk.

For non-Hodgkin lymphoma, careful, regular medical check-ups are important for people with known risk factors for non-Hodgkin lymphoma (such as HIV infections, organ transplants, autoimmune disease, or prior cancer treatment.

For breast cancer, the current recommendations include an annual MRI scan in addition to an annual mammogram for a subject at increased risk. Nonlimiting examples of chemoprevention include Tamoxifen, an oral selective antiestrogen agent (SERM) for estrogen receptor positive (ER+) breast cancer and the first chemoprevention drug to receive FDA approval and Raloxifene, another SERM which helps prevent breast cancer in postmenopausal women. Aromatase inhibitors or inactivators (AIs) reduce the incidence of new breast cancers in postmenopausal women who have an increased risk. Other treatments include hormone therapy - including antiestrogens, LH-RH agonists, aromatase inhibitors and SERMS Prophylactic treatment can include surgery, including, e.g., mastectomy and/or salpingo-oophorectomy.

For colorectal cancer, it is recommended that, beginning at age 50, both men and women at average risk for developing colorectal cancer should use one of the following screening tests: flexible sigmoidoscopy every 5 years*, colonoscopy every 10 years, double-contrast barium enema every 5 years*, CT colonography (virtual colonoscopy) every 5 years*, fecal occult blood test (FOBT) yearly, fecal immunochemical test (FIT) yearly (*if positive, colonoscopy should be done).

NSAIDS such as aspirin (acetylsalicylic acid; ASA) have been linked with reduced risk of polyps and colon cancer when used long-term. Mortality in regular users of ASA was about 40% lower for cancers of the colon and rectum. Daily or weekly nonaspirin (non-acetylsalicylic acid [non-ASA]) nonsteroidal anti-inflammatory drug (NSAID) use reduced 10-year incidence of proximal and distal colon cancer. Several observational studies have suggested a decreased risk of colon cancer among users of postmenopausal female hormone supplements. Celecoxib (Celebrex) is a member of a class of drugs known as COX-2 inhibitors. Some evidence suggests COX-2 drugs can reduce the risk of precancerous polyps in people who've been diagnosed with these polyps in the past. But COX-2 drugs carry a risk of heart problems, including heart attack. Two COX-2 inhibitor drugs were removed from the market because of these risks. Prophylactic treatment can include surgery to prevent colorectal cancer. For example, in certain inherited syndromes such as familial adenomatous polyposis, or inflammatory bowel disease such as ulcerative colitis, some or all of the colon and/or rectum in a subject can be removed to prevent colorectal cancer from occurring. Other prophylactic approaches can include bile acid reducing interventions and/or diet modification of less fat intake (e.g., 10% of dietary calories).

For lung cancer, the current recommendations include increased annual surveillance, which may include annual or regular low dose CT and/or chest X-ray until a subject reaches the age of 74. Prophylactic cranial irradiation (PCI) can be used as a treatment of subjects with small-cell lung cancer (SCLC) (e.g., in subjects that have achieved complete remission). A subject identified as having a genetic profile for increased incidence of lung cancer may be guided to take PCI treatment over others. Prophylactic treatment can include avoidance of and/or cessation of smoking and/or exposure to environmental elements associated with lung cancer.

For prostate cancer, the current recommendations include increased annual surveillance. Screening is recommended for men at higher risk with PSA screening with or without the digital rectal examination (DRE), which is recommended along with PSA for men with hypogonadism due to the reduced sensitivity of PSA. For men at higher risk with PSA <2.5 ng/mL, screening intervals can be extended to every 2 years (screening should be conducted yearly for men whose PSA level is 2.5 ng/mL or higher). Men at higher risk who have a PSA level of 4.0 ng/mL or higher have historically been referred for further evaluation or biopsy. GRS scores indicative of increased risk can be used to further aid medical professionals in these medical decisions.

Folate is a kind of vitamin B that occurs naturally in some foods, such as green vegetables, beans and orange juice. Folic acid is a man-made form of folate that is found in vitamin supplements and fortified foods, such as whole-grain breads and cereals. A 10-year study showed that the risk of prostate cancer was lower in men who had enough folate in their diets. However, the risk of prostate cancer was increased in men who took 1 milligram (mg) supplements of folic acid.

Finasteride and dutasteride are drugs used to lower the amount of male sex hormones made by the body. These drugs block the enzyme that changes testosterone into dihydrotesterone (DHT). Higher than normal levels of DHT may play a part in developing prostate cancer. Taking finasteride or dutasteride has been shown to lower the risk for prostate cancer, but it is not known if these drugs lower the risk of death from prostate cancer.

Some studies have shown that a diet high in lycopene may be linked to a decreased risk of prostate cancer, but other studies have not. It has not been proven that taking lycopene supplements decreases the risk of prostate cancer.

For ovarian cancer, current recommendations include counseling by a gynecologic oncologist and possibly testing to determine if the subject has a specific mutation associated with hereditary ovarian cancer syndrome. If so, the subject should receive annual rectovaginal pelvic examinations, (CA)-125 measurements and transvaginal ultrasound until childbearing is completed or at age 35, at which point prophylactic bilateral oophorectomy is recommended. Nonlimiting examples of prophylactic treatment can include prophylactic ovary removal, tubal ligation, salpingo-oophorectomy, ovarian ablation, vaccination [e.g., alpha-lactalbumin; WokVac triple antigen (HER2/neu, insulin-like growth factor binding protein-2 (IGFBP-2) and insulin-like growth factor receptor-1 (IGF1R); StemVac], oral contraceptives, multiple pregnancies, breast feeding, etc. To help reduce the risk of endometrial and ovarian cancer, some experts recommend discussing prophylactic hysterectomy and bilateral salpingo-oophorectomy with women older than 50 years who have hereditary nonpolyposis colorectal cancer (HNPCC).

For pancreatic cancer, genetic counseling and endoscopic ultrasound are possible screening protocols for a subject at increased risk. The goal of prophylactic surgery is to prevent malignant growth in patients with hereditary tumor predisposition. The pancreas presents as particularly challenging, due to the difficulty of operation and comparatively high risk of morbidity and even mortality. In addition, partial operative procedures and, more significantly, total resection lead to exocrine pancreas insufficiency and secondary diabetes, with grave consequences for the subject. Hereditary tumor predisposition syndromes that can result in pancreaticoduodenal endocrine tumors (PET) include multiple endocrine neoplasia type 1 syndrome and von Hippel-Lindau syndrome. As penetrance is maximally 70-80% and the 10-year survival rate over 80%, prophylactic pancreatic resection without evidence of a tumor is not indicated. However, prophylactic extension of a resection would be advised, should a PET be diagnosed. Subjects predisposed to developing ductal pancreatic carcinoma (PC) are at risk of familial pancreatic cancer syndrome (FPC), hereditary pancreatitis, and other hereditary tumor predisposition syndromes such as Peutz-Jeghers syndrome and familial atypical multiple mole-melanoma syndrome. As the gene defect responsible for FPC has yet to be identified and the penetrance of PC in the other tumor predisposition syndromes is low or unknown, a prophylactic pancreatectomy based on today's knowledge is not indicated. Prophylactic extension of the resection is advisable should PC or high-grade pancreatic intraepithelial neoplasia (PanIN) lesions be diagnosed, as these subjects often present with multifocal dysplasia and even carcinoma.

For testicular cancer, current recommendations include a testicular examination.

For melanoma, regular or more frequent examination by a dermatologist may be useful for a subject at increased risk.

For thyroid cancer, regular or more frequent examination of the thyroid may be useful for a subject at increased risk. A nonlimiting example of a preventative treatment can be prophylactic thyroidectomy.

For renal cancer, current recommendations include urinalysis, CT and/or MRI, which could be conducted regularly or more frequently in a subject at increased risk.

For glioma, diagnostic tests can be conducted upon presentation of symptoms characteristic of glioma.

For neuroblastoma, diagnostic tests can be conducted upon presentation of symptoms characteristic of neuroblastoma.

In some embodiments, the GRS calculated for a subject for a particular type of cancer can be used in combination with known clinical variables (e.g., for prostate cancer: prostate specific antigen (PSA), free to total PSA ratio, age, and/or family history) to predict a subject's risk of having or developing the particular type of cancer. This may help physicians and their patients decide whether to pursue screening and/or treatment protocols and to decide how aggressive such screening and/or treatment protocols can be or should be to be beneficial.

In carrying out the methods of this invention, detection reagents can be developed and used to identify a nucleic acid (e.g., an allele at a SNP site) of the present invention individually or in combination with the identification of other nucleic acids, and such detection reagents can be readily incorporated into one of the established kit or system formats that are well known in the art. The terms "kit" and "system," as used herein refer, e.g., to combinations of detection reagents, or one or more detection reagents in combination with one or more other types of elements or components (e.g., other types of biochemical reagents, containers, packages such as packaging intended for commercial sale, substrates to which detection reagents are attached, electronic hardware components, etc.)

Accordingly, the present invention provides a kit comprising reagents and instructions for carrying out the methods of this invention. In some embodiments, the present invention provides nucleic acid detection/identification kits and systems, including but not limited to, packaged probe and primer sets (e.g., TAQMAN probe/primer sets), arrays/microarrays of nucleic acid molecules, and/or beads that contain one or more probes, primers, or other detection reagents for detecting/identifying one or more nucleic acids of the present invention. The kits/systems can optionally include various electronic hardware components; for example, arrays ("DNA chips") and microfluidic systems ("lab-on-a-chip" systems) provided by various manufacturers. Other kits/systems (e.g., probe/primer sets) may not include electronic hardware components, but can be comprised of, for example, one or more detection reagents (along with, optionally, other biochemical reagents) packaged in one or more containers.

In some embodiments, a kit of this invention typically contains one or more detection reagents and other components (e.g., a buffer, enzymes such as DNA polymerases or ligases, chain extension nucleotides such as deoxynucleotide triphosphates, and in the case of Sanger-type DNA sequencing reactions, chain terminating nucleotides, positive control sequences, negative control sequences, etc.) necessary to carry out an assay or reaction, such as amplification and/or detection of a nucleic acid molecule of this invention. In some embodiments of the present invention, kits are provided that contain the necessary reagents to carry out one or more assays to detect one or more nucleic acids disclosed herein. In some embodiments of the present invention, allele detection kits/systems can be in the form of nucleic acid arrays, or compartmentalized kits, including microfluidic/lab-on-a-chip systems.

A detection kit/system of the present invention can include components that are used to prepare nucleic acids from a test sample for the subsequent amplification and/or detection of a nucleic acid molecule of this invention, as well as for the detection and/or quantitation of a polypeptide or peptide of this invention. Such sample preparation components can be used to produce, e.g., nucleic acid extracts (including DNA and/or RNA), proteins, protein fractions, cellular fractions and/or membrane extracts from any bodily fluids or materials (such as blood, serum, plasma, urine, saliva, phlegm, sputum, joint fluids, fecal material, secretions, gastric juices, semen, tears, sweat, spinal fluid, etc.), skin, hair, cells (especially nucleated cells), biopsies, washes, lavages, exudates, buccal swabs and/or tissue specimens. The test samples used in the above-described methods will vary based on such factors as the assay format, nature of the detection method, and the specific tissues, cells or extracts used as the test sample to be assayed. Methods of preparing nucleic acids, proteins, and cell extracts are well known in the art and can be readily adapted to obtain a sample that is compatible with the system utilized. Automated sample preparation systems for extracting nucleic acids from a test sample are commercially available (e.g., Qiagen's BIOROBOT 9600, Applied Biosystems' PRISM 6700, and Roche Molecular Systems COBAS AmpliPrep System).

Another form of kit included in the present invention is a compartmentalized kit. A compartmentalized kit includes any kit in which reagents are contained in separate containers. Such containers include, for example, small glass containers, plastic containers, strips of plastic, glass or paper, or arraying material such as silica. Such containers allow one to efficiently transfer reagents from one compartment to another compartment such that the test samples and reagents are not cross-contaminated, or from one container to another vessel not included in the kit, and the agents or solutions of each container can be added in a quantitative fashion from one compartment to another or to another vessel. Such containers may include, for example, one or more containers which will accept the test sample, one or more containers which contain at least one detection reagent for detecting one or more nucleic acids of the present invention, one or more containers which contain wash reagents (such as phosphate buffered saline, Tris-buffers, etc.), and one or more containers which contain the reagents used to reveal the presence of the bound nucleic acid or other detection reagents. The kit can optionally further comprise compartments and/or reagents for, for example, nucleic acid amplification or other enzymatic reactions such as primer extension reactions, hybridization, ligation, electrophoresis (e.g., capillary electrophoresis), mass spectrometry, and/or laser-induced fluorescence detection. The kit can also include instructions for using the kit. Exemplary compartmentalized kits include microfluidic devices known in the art (e.g., Weigl et al. (2003) "Lab-on-a-chip for drug development" Adv Drug Deliv Rev. 55(3):349-77). In such microfluidic devices, the containers may be referred to as, for example, microfluidic "compartments," "chambers," or "channels."

A kit of this invention can further comprise therapeutic agents and/or compositions that can be used, for example in a treatment protocol and/or prophylactic treatment protocol for a subject identified according to the methods described herein as a subject having an increased risk of having or developing a type of cancer described herein. As used herein, a "prophylactic treatment" describes the use of medication and/or other intervention and/or other therapy before the clinical manifestation of the disease or disorder.

The present invention also provides a computer program product comprising: a computer readable storage medium having computer readable code embodied in the medium, the computer code comprising: computer readable code to perform operations to carry out the methods of this invention.

Further provided herein is a computer system, comprising: a processor; and a memory coupled to the processor, the memory comprising computer readable program code embodied therein that, when executed by the processor, causes the processor to perform operations to carry out the methods of this invention.

As noted above, a kit of this invention can comprise electronic hardware components. In some embodiments of this invention, the electronic hardware may perform and/or support functionality that corresponding to various operations described herein. For example, functions described and/or illustrated in diagrams and/or flowchart illustrations of methods, apparatus (systems) and/or computer program products according to some embodiments may be performed by the electronic hardware. It is understood that each block of the block diagrams and/or flowchart illustrations, and combinations of blocks in the block diagrams and/or flowchart illustrations, can be implemented by computer program instructions. These computer program instructions may be provided to a processor of a general purpose computer, special purpose computer, and/or other programmable data processing apparatus to produce a machine, such that the instructions, which execute via the processor of the computer and/or other programmable data processing apparatus, create means for implementing the functions/acts specified in the block diagrams and/or flowchart block or blocks.

These computer program instructions may also be stored in a computer-readable memory that can direct a computer or other programmable data processing apparatus to function in a particular manner, such that the instructions stored in the computer-readable memory produce an article of manufacture including instructions which implement the function/act specified in the block diagrams and/or flowchart block or blocks.

The computer program instructions may also be loaded onto a computer or other programmable data processing apparatus to cause a series of operational steps to be performed on the computer or other programmable apparatus to produce a computer-implemented process such that the instructions which execute on the computer or other programmable apparatus provide steps for implementing the functions/acts specified in the block diagrams and/or flowchart block or blocks.

Accordingly, the present invention may be embodied in hardware and/or in software (including firmware, resident software, micro-code, etc.). Furthermore, embodiments of the present invention may take the form of a computer program product on a computer-usable or computer-readable non-transient storage medium having computer-usable or computer-readable program code embodied in the medium for use by or in connection with an instruction execution system,

The computer-usable or computer-readable medium may be, for example but not limited to, an electronic, optical, electromagnetic, infrared, or semiconductor system, apparatus, or device. More specific examples (a non-exhaustive list) of the computer-readable medium would include the following: an electrical connection having one or more wires, a portable computer diskette, a random access memory (RAM), a read-only memory (ROM), an erasable programmable read-only memory (EPROM or Flash memory), an optical fiber, and a portable compact disc read-only memory (CD-ROM).

Computer program code for carrying out operations for aspects of the present disclosure may be written in any combination of one or more programming languages, including an object oriented programming language such as Java, Scala, Smalltalk, Eiffel, JADE, Emerald, C++, C#, VB.NET, Python or the like, conventional procedural programming languages, such as the "C" programming language, Visual Basic, Fortran 2003, Perl, COBOL 2002, PHP, ABAP, dynamic programming languages such as Python, Ruby and Groovy, or other programming languages. The program code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider) or in a cloud computer environment or offered as a service such as a Software as a Service (SaaS).

### Definitions

As used herein, "a," "an" or "the" can mean one or more than one. For example, "a" cell can mean a single cell or a multiplicity of cells.

Also as used herein, "and/or" refers to and encompasses any and all possible combinations of one or more of the associated listed items, as well as the lack of combinations when interpreted in the alternative ("or").

Furthermore, the term "about," as used herein when referring to a measurable value such as an amount of a compound or agent of this invention, dose, time, temperature, and the like, is meant to encompass variations of ± 20%, ± 10%, ± 5%, ± 1%, ± 0.5%, or even ± 0.1% of the specified amount.

All the SNP positions described herein are based on Build 36.

Also as used herein, "linked" describes a region of a chromosome that is shared more frequently in family members or members of a population manifesting a particular phenotype and/or affected by a particular disease or disorder, than would be expected or observed by chance, thereby indicating that the gene or genes or other identified marker(s) within the linked chromosome region contain or are associated with an allele that is correlated with the phenotype and/or presence of a disease or disorder (e.g., aggressive PCa), or with an increased or decreased likelihood of the phenotype and/or of the disease or disorder. Once linkage is established, association studies can be used to narrow the region of interest or to identify the marker (e.g., allele or haplotype) correlated with the phenotype and/or disease or disorder.

Furthermore, as used herein, the term "linkage disequilibrium" or "LD" refers to the occurrence in a population of two or more (e.g., 3, 4, 5, 6, 7, 8, 9, 10, etc.) linked alleles at a frequency higher or lower than expected on the basis of the gene frequencies of the individual genes. Thus, linkage disequilibrium describes a situation where alleles occur together more often than can be accounted for by chance, which indicates that the two or more alleles are physically close on a DNA strand.

The term "genetic marker" or "polymorphism" as used herein refers to a characteristic of a nucleotide sequence (e.g., in a chromosome) that is identifiable due to its variability among different subjects (i.e., the genetic marker or polymorphism can be a single nucleotide polymorphism, a restriction fragment length polymorphism, a microsatellite, a deletion of nucleotides, an addition of nucleotides, a substitution of nucleotides, a repeat or duplication of nucleotides, a translocation of nucleotides, and/or an aberrant or alternate splice site resulting in production of a truncated or extended form of a protein, etc., as would be well known to one of ordinary skill in the art).

A "single nucleotide polymorphism" (SNP) in a nucleotide sequence is a genetic marker that is polymorphic for two (or in some case three or four) alleles. SNPs can be present within a coding sequence of a gene, within noncoding regions of a gene and/or in an intergenic (e.g., intron) region of a gene. A SNP in a coding region in which both forms lead to the same polypeptide sequence is termed synonymous (i.e., a silent mutation) and if a different polypeptide sequence is produced, the alleles of that SNP are non-synonymous. SNPs that are not in protein coding regions can still have effects on gene splicing, transcription factor binding and/or the sequence of non-coding RNA.

The SNP nomenclature provided herein refers to the official Reference SNP (rs) identification number as assigned to each unique SNP by the National Center for Biotechnological Information (NCBI), which is available in the GenBank® database.

In some embodiments, the term genetic marker is also intended to describe a phenotypic effect of an allele or haplotype, including for example, an increased or decreased amount of a messenger RNA, an increased or decreased amount of protein, an increase or decrease in the copy number of a gene, production of a defective protein, tissue or organ, etc., as would be well known to one of ordinary skill in the art.

An "allele" as used herein refers to one of two or more alternative forms of a nucleotide sequence at a given position (locus) on a chromosome. An allele can be a nucleotide present in a nucleotide sequence that makes up the coding sequence of a gene and/or an allele can be a nucleotide in a non-coding region of a gene (e.g., in a genomic sequence). A subject's genotype for a given gene is the set of alleles the subject happens to possess. As noted herein, an individual can be heterozygous or homozygous for any allele of this invention.

Also as used herein, a "haplotype" is a set of alleles on a single chromatid that are statistically associated. It is thought that these associations, and the identification of a few alleles of a haplotype block, can unambiguously identify all other alleles in its region. The term "haplotype" is also commonly used to describe the genetic constitution of individuals with respect to one member of a pair of allelic genes; sets of single alleles or closely linked genes that tend to be inherited together.

The terms "increased risk" and "decreased risk" as used herein define the level of risk that a subject has of developing a particular cancer, as compared to a control (e.g., a subject or a population of subjects; i.e., a general population) that does not have the polymorphisms and alleles of this invention in the control nucleic acid.

A sample of this invention can be any sample containing nucleic acid of a subject, as would be well known to one of ordinary skill in the art. Nonlimiting examples of a sample of this invention include a cell, a body fluid, a tissue, biopsy material, a washing, a swabbing, etc., as would be well known in the art.

A subject of this invention is any animal that is susceptible to any of the cancers as defined herein and can include, for example, humans, as well as animal models of cancer (e.g., rats, mice, dogs, nonhuman primates, etc.). In some aspects of this invention, the subject can be Caucasian (e.g., white; European-American; Hispanic), as well as of black African ancestry (e.g., black; African American; African-European; African-Caribbean, etc.) or Asian. In further aspects of this invention, the subject can have a family history of a particular cancer (e.g., having at least one first degree relative having or diagnosed with the cancer) and in some embodiments, the subject does not have or does not have knowledge of a family history of the particular cancer. Additionally a subject of this invention can have a diagnosis of a particular cancer in certain embodiments and in other embodiments, a subject of this invention does not have a diagnosis of a particular cancer.

As used herein, "nucleic acid" encompasses both RNA and DNA, including cDNA, genomic DNA, mRNA, synthetic (e.g., chemically synthesized) DNA and chimeras, fusions and/or hybrids of RNA and DNA. The nucleic acid can be double-stranded or single-stranded. Where single-stranded, the nucleic acid can be a sense strand or an antisense strand. In some embodiments, the nucleic acid can be synthesized using oligonucleotide analogs or derivatives (e.g., inosine or phosphorothioate nucleotides, etc.). Such oligonucleotides can be used, for example, to prepare nucleic acids that have altered base-pairing abilities or increased resistance to nucleases.

An "isolated nucleic acid" is a nucleotide sequence that is not immediately contiguous with nucleotide sequences with which it is immediately contiguous (one on the 5' end and one on the 3' end) in the naturally occurring genome of the organism from which it is derived or in which it is detected or identified. Thus, in one embodiment, an isolated nucleic acid includes some or all of the 5' non-coding (e.g., promoter) sequences that are immediately contiguous to a coding sequence. The term therefore includes, for example, a recombinant DNA that is incorporated into a vector, into an autonomously replicating plasmid or virus, or into the genomic DNA of a prokaryote or eukaryote, or which exists as a separate molecule (e.g., a cDNA or a genomic DNA fragment produced by PCR or restriction endonuclease treatment), independent of other sequences. It also includes a recombinant DNA that is part of a hybrid nucleic acid encoding an additional polypeptide or peptide sequence.

The term "isolated" can refer to a nucleic acid or polypeptide that is substantially free of cellular material, viral material, and/or culture medium (e.g., when produced by recombinant DNA techniques), or chemical precursors or other chemicals (when chemically synthesized). Moreover, an "isolated fragment" is a fragment of a nucleic acid or polypeptide that is not naturally occurring as a fragment and would not be found in the natural state.

The term "oligonucleotide" refers to a nucleic acid sequence of at least about five nucleotides to about 500 nucleotides (e.g. 5, 6, 7, 8, 9, 10, 12, 15, 18, 20, 21, 22, 25, 30, 35, 40, 45, 50, 55, 60, 65, 70, 75, 80, 85, 90, 100, 125, 150, 175, 200, 250, 300, 350, 400, 450 or 500 nucleotides). In some embodiments, for example, an oligonucleotide can be from about 15 nucleotides to about 30 nucleotides, or about 20 nucleotides to about 25 nucleotides, which can be used, for example, as a primer in a polymerase chain reaction (PCR) amplification assay and/or as a probe in a hybridization assay or in a microarray. Oligonucleotides of this invention can be natural or synthetic, e.g., DNA, RNA, PNA, LNA, modified backbones, etc., as are well known in the art.

The present invention further provides fragments of the nucleic acids of this invention, which can be used, for example, as primers and/or probes. Such fragments or oligonucleotides can be detectably labeled or modified, for example, to include and/or incorporate a restriction enzyme cleavage site when employed as a primer in an amplification (e.g., PCR) assay.

The detection of a polymorphism, genetic marker or allele of this invention can be carried out according to various protocols standard in the art and as described herein for analyzing nucleic acid samples and nucleotide sequences, as well as identifying specific nucleotides in a nucleotide sequence.

For example, nucleic acid can be obtained from any suitable sample from the subject that will contain nucleic acid and the nucleic acid can then be prepared and analyzed according to well-established protocols for the presence of genetic markers according to the methods of this invention. In some embodiments, analysis of the nucleic acid can be carried by amplification of the region of interest according to amplification protocols well known in the art (e.g., polymerase chain reaction, ligase chain reaction, strand displacement amplification, transcription-based amplification, self-sustained sequence replication (3SR), Qβ replicase protocols, nucleic acid sequence-based amplification (NASBA), repair chain reaction (RCR) and boomerang DNA amplification (BDA), etc.). The amplification product can then be visualized directly in a gel by staining or the product can be detected by hybridization with a detectable probe. When amplification conditions allow for amplification of all allelic types of a genetic marker, the types can be distinguished by a variety of well-known methods, such as hybridization with an allele-specific probe, secondary amplification with allele-specific primers, by restriction endonuclease digestion, and/or by electrophoresis. Thus, the present invention further provides oligonucleotides for use as primers and/or probes for detecting and/or identifying genetic markers according to the methods of this invention.

In some embodiments of this invention, detection of an allele or combination of alleles of this invention can be carried out by an amplification reaction and single base extension. In particular embodiments, the product of the amplification reaction and single base extension is spotted on a silicone chip.

In yet additional embodiments, detection of an allele or combination of alleles of this invention can be carried out by matrix-assisted laser desorption/ionization-time of flight mass spectrometry (MALDI-TOF-MS).

It is further contemplated that the detection of an allele or combination of alleles of this invention can be carried out by various methods that are well known in the art, including, but not limited to nucleic acid sequencing, hybridization assay, restriction endonuclease digestion analysis, electrophoresis, and any combination thereof.

The present invention further comprises a kit or kits to carry out the methods of this invention. A kit of this invention can comprise reagents, buffers, and apparatus for mixing, measuring, sorting, labeling, etc., as well as instructions and the like as would be appropriate for genotyping any combination of, or all of, the SNPs of Tables 1-17 in a nucleic acid sample. The kit may further comprise control reagents, e.g., to identify markers for a specific ethnicity or gender.

The present invention is more particularly described in the following examples that are intended as illustrative only since numerous modifications and variations therein will be apparent to those skilled in the art.

### EXAMPLES

### Example 1. Revisit of risk-associated SNPs: genetic risk score is effective in identifying high-risk subjects

Genome-wide association studies (GWAS) have identified many single nucleotide polymorphisms (SNPs) that are associated with risk for complex diseases since 2007. Due to its rigorous study design and statistical criteria, most of these SNPs represent true associations and can be consistently replicated in independent study populations. Although the individual effects of these SNPs on disease risk are modest, their cumulative effect is stronger and is typically greater than family history, a well-accepted measurement for familial risk. Nevertheless, it is widely perceived that these SNPs have little clinical utility. This perception is primarily based on the modest values of AUC (area under the receiver operating characteristic curve) of these SNPs.

AUC is a commonly used statistic to measure the ability of a marker to discriminate patients from non-patients. If the distributions of a marker in patients and non-patients completely separate or overlap, AUC of the marker would be 100% (maximum) or 50% (minimum), respectively. Therefore, AUC is appropriate for assessing the performance of diagnostic markers because their intended use is to distinguish patients from non-patients. AUC, however, is not appropriate for assessing the performance of predictive markers because they are not intended to distinguish patients from non-patients but to identify high-risk individuals. For the latter purpose, a more appropriate statistic for measuring the performance is the positive predictive value (PPV), i.e., the likelihood of being diagnosed with a disease among high-risk subjects defined by a marker. For this reason, AUC is not used to judge the performance of family history in identifying high-risk individuals; otherwise, it would not be widely adopted due to a poor AUC (typically <55%) for most complex diseases such as cancers.

In this study, we estimated and compared PPVs of SNPs and family history for identifying high-risk individuals by re-analyzing the data from two studies on breast (BrCa) and prostate cancer (PCa). In the first study, the AUC of 10 BrCa risk-associated SNPs and a model with four known BrCa risk factors (including family history) were estimated in 5,590 case subjects and 5,998 control subjects from four prospective studies and one case-control study. AUC was 58.0%, 59.7%, and 61.8% for the four known risk factors, 10 BrCa risk-associated SNPs, and a combination of both, respectively. Based primarily on these AUC findings, it was widely misinterpreted that SNPs provided little additional information to known risk factors for predicting BrCa risk. We re-analyzed these data and calculated PPV of 10 SNPs and family history for predicting a BrCa diagnosis. In the entire cohort, the percentage of BrCa patients was 48.2%. In women with a strong family history (≥2 first-degree BrCa relatives, representing 1.6% of women in the study), the PPV was 58.3% (95% Confidence Interval [CI]: 58.1-58.6%) (**Fig. 1A**)**.** In comparison, if women with ≥13 risk alleles of these 10 risk SNPs were considered as high risk (representing 5.5% of women in the study), the PPV was 63.0% (95% CI: 62.9-63.1%) (**Fig. 1B**)**.** These results indicate that SNPs are more effective to identify high-risk women than the widely accepted predictive marker of family history.

In the prostate cancer study, the subjects were 1,654 men from the placebo arm of the randomized Reduction by Dutasteride of Prostate Cancer Events (REDUCE) trial. All men had an initial negative prostate biopsy and underwent study-mandated biopsies at year 2 and 4. The AUC of family history and genetic risk score (GRS) based on 33 established PCa risk-associated SNPs was 52% and 59%, respectively. We re-analyzed theses data to estimate the likelihood of being diagnosed among high-risk men defined by family history and GRS. The PPV was 31.8% (95% CI: 31.6-32.0%) for the 12.9% men with a positive family history of PCa (**Fig. 1C**). In contrast, the PPV was 36.1% (95% CI: 35.9-36.4%) for the 10.7% men with a GRS >=2.0 (**Fig. 1D**). Importantly, even in men with a negative family history, GRS was a significant predictor of PCa risk; the PPV was 33.3% (95% CI: 33.1-33.6%) for the 9.3% men with a negative family history but GRS >=2.0, higher than that of men with a positive family history (**Fig. 1E**)**.** If either a positive family history or a GRS >=2.0 was considered as high-risk (representing 22.2% men in the study), the PPV was 32.4% (95% CI: 32.3-32.6) (**Fig. 1F**). It is also noted that PPV for Gleason scores of 7 or greater in PCa were higher for men with a positive family history and/or higher GRS.

Identifying high-risk subjects has potential clinical utilities, particularly for targeted cancer screening. Cancer screening is intended to identify asymptomatic cancer at early and treatable stages; however, it is also associated with potential harms such as false positives of screenings and detection of cancer where aggressive treatment is unnecessary. Evidence-based studies suggest that the net benefit of the current one-size-fits-all cancer screening strategy, measured by mortality, quality of life, and cost, is modest for several other types of cancer. Targeted cancer screening among high-risk subjects may tip the balance towards greater benefits. Currently, a positive family history is commonly used to define high-risk subjects. Based on the data described above, it is reasonable that GRS be incorporated with family history in better identifying high-risk subjects for cancer screening. For example, high-risk subjects can be defined as either with a positive family history or a GRS >=2.0.

This study provides evidence that GRS is effective and performs better than family history in identifying high-risk or increased risk individuals. In summary, GRS calculated from multiple disease risk-associated SNPs is effective in identifying increased risk subjects for most complex diseases.

### Example 2. Genetic risk score: 21^{st} century family history

Family history (FH) of cancer is widely accepted by patients and physicians for assessing individual cancer risk. Occurrence of cancer in family members is a major motivation for patients to seek cancer screening. Primary care physicians typically collect FH information from their patients to develop corresponding cancer screening strategies. Various clinical guidelines also incorporate FH information to determine the timing and frequency of cancer screening.

A great advantage of FH is that it can be obtained without a laboratory test. In addition, FH captures both genetic and shared life styles among relatives. However, FH is only an indirect measurement of risks via relatives and therefore has many inherent limitations. FH information depends on family structure, mortality from other competing diseases among relatives, levels of communication, and historical incidences of specific cancers in the population. FH status can change qualitatively, from negative to positive, and quantitatively, from fewer to more affected family members. The magnitude of risk associated with FH is also difficult to estimate and is often overestimated in case-control studies due to differential recall bias among subjects with or without cancer. For instance, the relative risk (RR) of a positive FH for prostate cancer was estimated at 2.5 from a large meta-analysis. However, the estimate of RR was lower and variable (between 1.31 and 1.91) from several prospective studies where there was no differential recall bias. Another limitation of FH is that it cannot distinguish risk among siblings, even though they share 50% of their genes on average.

A genetic risk score (GRS), which is calculated based on multiple cancer risk-associated SNPs, has been proposed as a measurement for inherited risk. In some embodiments, GRS can be used to supplement FH to better define a subject's cancer risk. GRS can be especially informative for subjects without a FH. It is calculated from multiple cancer risk-associated single nucleotide polymorphisms (SNPs) implicated in genome-wide association studies (GWAS). Because GRS is based on genotypes of individuals themselves, it is a direct, objective, and truly individualized measurement of inherited risk, which does not change over time. A GRS can be incorporated into a subject's primary care.

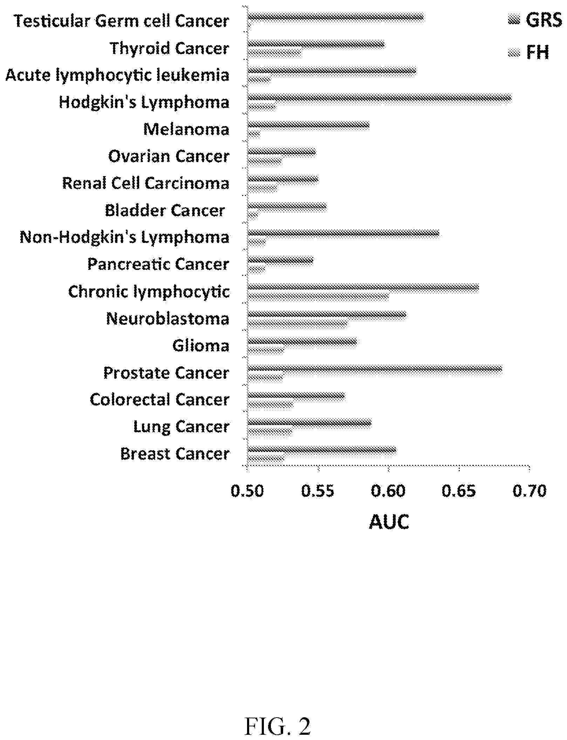

In a head-to-head comparison of FH and GRS in discriminating risk for prostate cancer among five study populations, GRS was consistently shown to have a better discriminative performance. The better performance of GRS over FH was also demonstrated in other common cancers. In a study that compared the area under the receiver operating characteristic curve (AUC) of FH and GRS in discriminating seven common cancers (breast, prostate, colorectal, ovary, bladder, glioma, and pancreas), GRS outperformed FH in each of these cancers (**Fig. 2**)**.**

To further assess the generalizability of these findings in other sites of cancer, we systematically calculated AUC of FH and GRS in discriminating risk for cancer in which at least three risk-associated SNPs have been identified. Only SNP associations that reached a genome-wide significance level (*P*<5x10⁻⁸) in the combined analysis and were independent (*r*² <0.2 for linkage disequilibrium measurement between SNPs) were selected. For FH, the prevalence and RR of FH for each cancer was obtained from the largest prospective study; otherwise, from the largest case-control study. The genetic variance explained by FH and risk-associated SNPs, as well as the AUC in predicting cancer, was calculated for each type of cancer.

As shown in **Table 18,** compared to FH, GRS accounted for more genetic variance and had a higher AUC for each of these 17 sites of cancer. Using breast cancer as an example, FH accounted for 0.8% of total genetic variance. In contrast, GRS calculated from 67 established breast cancer risk-associated SNPs accounted for 14.3% total genetic variance. The AUC of FH and GRS for predicting breast cancer was 0.526 and 0.605, respectively. These results suggest that GRS is a better predictor of cancer risk than FH and it adds value to FH in predicting cancer risk.

Despite the consistent findings to date, several barriers have been encountered in translating GRS to clinics and populations. One of the most cited arguments against its clinical use is the low AUC value of GRS and the marginal improvement of AUC over existing diagnostic markers. The fallacy of this argument is that it mixes two different types of predictors; one measures the likelihood of developing cancer and the other measures the occurrence of cancer. The first type of predictors, including FH and GRS, identifies high-risk subjects for further evaluation using the second type of predictors. For this reason, different criteria should be used to assess the performance for these two types of predictors. In fact, we correctly apply this principle for assessing the performance of FH and widely accept FH as a predictor for high-risk individuals even though its AUC is modest (typically < 0.55). Similarly, whether GRS is an acceptable predictor of high-risk individuals should be judged based on its comparative effectiveness with FH, not other diagnostic markers.

Another argument against GRS is that measurement of cancer-risk-associated SNPs may lead to potential worry and anxiety. This concern also comes from misunderstanding GRS. It is important to note that a higher GRS, like having a positive FH, only suggests an increased risk over the general population. Efforts should be made to educate physicians and patients about the clinical utility and interpretation of GRS, not to discard it simply because results may be misinterpreted.

The third argument against the use of GRS at the current time is that more cancer risk-associated SNPs are expected to be discovered by future and larger GWAS. However, further improvements in the discriminative performance of GRS with more SNPs may be limited because the effects of yet-to-be discovered SNPs are likely to be smaller. A plateau effect in AUC with an increasing number of risk-associated SNPs has been predicted and observed.¹⁰ Therefore, the use of GRS based on those already discovered risk-associated SNPs is justified.

Differing from a questionnaire-based FH, the measurement of GRS requires DNA samples and incurs costs. Fortunately, DNA can be easily obtained from cells in peripheral blood or saliva. Current high-throughput genotyping technology can measure these hundreds of cancer risk-associated SNPs in a single assay within hours. The expenditure of genotyping is modest now (<$50) and will continue to decrease. However, formal cost-effectiveness analysis is needed to assess the balance of direct and indirect benefits resulting from the improved risk assessment of GRS while considering the cost of SNP genotyping.

One utility is for primary care physicians to use GRS and traditional FH to determine which, when, and how often their patients should screen for specific cancer. This is particularly relevant considering that current one-size-fits-all cancer screening is often ineffective for many types of cancer by the U.S. Preventive Services Task Force. Future efforts should be devoted to assessing the effect of genomic-targeted screening on reduction of mortality, as well as to evaluate its cost-effectiveness, to understand its impact on cancer screening behaviors, and the ethical, legal, and social implications.

### Example 3. Calculation of genetic risk score (GRS)

As one nonlimiting example, a subject is identified according to the methods described herein as having the following genotype for the alleles defined herein as markers for pancreatic cancer (Table 8):
SNP rs3790844 (allelic OR=1.3) homozygous for risk allele A
SNP rs505922 (allelic OR = 1.2) homozygous for risk allele C
SNP rs9453325 (allelic OR = 1.26) heterozygous for allele C

The calculation of the genetic risk score would be:
For SNP rs3790844:
   1) Allelic OR = 1.3
   2) Genotypic ORs for A/A = 1.3 x 1.3 = 1.69, A/G = 1.3, and G/G = 1.0 and Genotypic Frequencies of A/A, A/G and G/G are 0.61, 0.35 and 0.04, for CEU population (hapmap.org), respectively
   3) Relative Risk to Average Risk in the population for each genotype
      a. A/A = 1.69/ (1.69^{∗} 0.61 + 1.3 ^{∗} 0.35 + 1 ^{∗} 0.04) = 1.10
      b. A/G = 1.3/ (1.69^{∗} 0.61 + 1.3 ^{∗} 0.35 + 1 ^{∗} 0.04) = 0.85
      c. G/G= 1 / (1.69^{∗} 0.61 + 1.3 ^{∗} 0.35 + 1 ^{∗} 0.04) = 0.65
For SNP rs505922
   1) Allelic OR = 1.2
   2) Genotypic ORs for C/C = 1.2 x 1.2 = 1.44, C/T = 1.2, and T/T = 1.0 and Genotypic Frequencies of C/C, C/T and T/T are 0.12, 0.48 and 0.40 for CEU population (hapmap.org), respectively
   3) Relative Risk to Average Risk in the population for each genotype
      a. C/C = 1.44/ (1.44^{∗} 0.12 + 1.2 ^{∗} 0.48 + 1 ^{∗} 0.40) = 1.25
      b. C/T = 1.2/(1.44^{∗} 0.12 + 1.2 ^{∗} 0.48 + 1 ^{∗} 0.40) = 1.04
      c. T/T = 1 / (1.44^{∗} 0.12 + 1.2 ^{∗} 0.48 + 1 ^{∗} 0.40)= 0.87
For SNP rs9453325
   1) Allelic OR = 1.26
   2) Genotypic ORs for C/C = 1.26 x 1.26 = 1.59, C/T = 1.26, and T/T = 1.0 and Genotypic Frequencies of C/C, C/T and T/T are 0.06, 0.50 and 0.44, for CEU population (hapmap.org), respectively
   3) Relative Risk to the Average Risk in the population for each genotype
      a. C/C = 1.59/ (1.59^{∗} 0.06 + 1.26 ^{∗} 0.50 + 1 ^{∗} 0.44) = 1.36
      b. C/T = 1.3/ (1.59^{∗} 0.06 + 1.26 ^{∗} 0.50 + 1 ^{∗} 0.44) = 1.08
      c. T/T = 1 / (1.59^{∗} 0.06 + 1.26 ^{∗} 0.50 + 1 ^{∗} 0.44) = 0.85
   4) The calculation of the genetic risk score would be: 1.10 X 1.25 X 1.08 = 1.49.
      a. If the subject is heterozygous for the risk allele at all three SNP locations, the calculation of the genetic risk score would be: 0.85 X 1.04 X 1.08 = 0.95.

As a further nonlimiting example, using a similar method, a risk score can also be calculated for individuals with family history of pancreatic cancer and individuals without family history of pancreatic cancer using the prevalence (2%) and relative risk (RR = 1.62) for pancreatic cancer (Table 18).

For individuals with family history of pancreatic cancer, the risk relative to the average risk = 1.62/ (1.62^{∗} 0.02 + 1 ^{∗} 0.98) = 1.60. For individuals without family history of pancreatic cancer, the risk relative to the average risk = 1.0/ (1.62^{∗} 0.02 + 1 ^{∗} 0.98) = 0.99.

The foregoing is illustrative of the present invention, and is not to be construed as limiting thereof. The invention is defined by the claims provided herein, with equivalents of the claims to be included therein.

All publications, patent applications, patents, patent publications, sequences identified by GenBank® Database accession numbers and/or SNP accession numbers, and other references cited herein are incorporated by reference in their entireties for the teachings relevant to the sentence and/or paragraph in which the reference is presented.

**Table 1. Breast Cancer**

| SNP | Chromosome (CHR) | Risk Allele | Odds Ratio (OR)/Risk |
|---|---|---|---|
| rs616488 | 1 | A | 1.06 |
| rs11552449 | 1 | T | 1.08 |
| rs4849887 | 2 | C | 1.11 |
| rs2016394 | 2 | G | 1.05 |
| rs1550623 | 2 | G | 1.1 |
| rs16857609 | 2 | T | 1.09 |
| rs6762644 | 3 | G | 1.06 |
| rs12493607 | 3 | C (minor) | 1.04 |
| rs9790517 | 4 | T | 1.09 |
| rs6828523 | 4 | C | 1.12 |
| rs10472076 | 5 | C | 1.06 |
| rs1353747 | 5 | T | 1.11 |
| rs14.32679 | 5 | C | 1.06 |
| rs11242675 | 6 | T | 1.03 |
| rs204247 | 6 | G | 1.06 |
| rs720475 | 7 | G | 1.08 |
| rs9693444 | 8 | A | 1.07 |
| rs6472903 | 8 | T | 1.14 |
| rs2943559 | 8 | G | 1.17 |
| rs11780156 | 8 | T | 1.13 |
| rs10759243 | 9 | A | 1.07 |
| rs7072776 | 10 | A | 1.11 |
| rs11814448 | 10 | C | 1.35 |
| rs7904519 | 10 | G | 1.06 |
| rs11199914 | 10 | C | 1.06 |
| rs3903072 | 11 | G | 1.09 |
| rs11820646 | 11 | C | 1.08 |
| rs12422552 | 12 | C (minor) | 1.11 |
| rs17356907 | 12 | A | 1.12 |
| rs11571833 | 13 | T (minor) | 1.39 |
| rs2236007 | 14 | G | 1.14 |
| rs2588809 | 14 | T | 1.07 |
| rs941764 | 14 | G | 1.05 |
| rs17817449 | 16 | T | 1.05 |
| rs13329835 | 16 | G | 1.14 |
| rs527616 | 18 | G (major) | 1.1 |
| rs1436904 | 18 | T | 1.08 |
| rs4808801 | 19 | A | 1.06 |
| rs3760982 | 19 | A | 1.06 |
| rs132390 | 22 | C | 1.36 |
| rs6001930 | 22 | C | 1.17 |
| rs11249433 | 1 | G | 1.09 |
| rs13387042 | 2 | A | 1.14 |
| rsl045485 | 2 | G (major) | 1.03 |
| rs4973768 | 3 | T | 1.1 |
| rs10069690 | 5 | T | 1.06 |
| rs10941679 | 5 | G | 1.13 |
| rs889312 | 5 | C | 1.12 |
| rs17530068 | 6 | G | 1.05 |
| rs3757318 | 6 | A | 1.16 |
| rs2046210 | 6 | A | 1.08 |
| rs13281615 | 8 | G | 1.09 |
| rs1011970 | 9 | T | 1.06 |
| rs865686 | 9 | T | 1.12 |
| rs2380205 | 10 | C | 1.02 |
| rs10995190 | 10 | G | 1.16 |
| rs704010 | 10 | T | 1.08 |
| rs2981579 | 10 | A | 1.27 |
| rs3817198 | 11 | C | 1.07 |
| rs614367 | 11 | T | 1.21 |
| rs10771399 | 12 | A | 1.16 |
| rs1292011 | 12 | A | 1.09 |
| rs999737 | 14 | C | 1.09 |
| rs3803662 | 16 | A | 1.24 |
| rs6504950 | 17 | G | 1.06 |
| rs8170 | 19 | G | 1.04 |
| rs2823093 | 21 | G | 1.09 |

**Table 2. Lung Cancer**

| SNP | CHR | Risk Allele | OR/Risk |
|---|---|---|---|
| rs4975616 | 5 | A | 1.15 |
| rs3117582 | 6 | G | 1.24 |
| rs8034191 | 15 | C | 1.29 |
| rs6489769 | 12 | C | 1.2 |

**Table 3. Colorectal Cancer**

| SNP | CHR | Risk Allele | OR/Risk |
|---|---|---|---|
| rs6691170 | 1 | *T* | 1.06 |
| rs6687758 | 1 | *G* | 1.09 |
| rs11903757 | 2 | C | 1.16 |
| rs10936599 | 3 | *C* | 1.08 |
| rs647161 | 5 | A | 1.07 |
| rs1321311 | 6 | *A* | 1.1 |
| rs16892766 | 8 | *C* | 1.25 |
| rs7014346 | 8 | *A* | 1.19 |
| rs10795668 | 10 | *G* | 1.12 |
| rs3824999 | 11 | *G* | 1.08 |
| rs3802842 | 11 | *C* | 1.11 |
| rs10774214 | 12 | T | 1.04 |
| rs7136702 | 12 | *T* | 1.06 |
| rs11169552 | 12 | *C* | 1.09 |
| rs4444235 | 14 | *C* | 1.09 |
| rs1957636 | 14 | *T* | 1.08 |
| rs4779584 | 15 | *T* | 1.15 |
| rs11632715 | 15 | *A* | 1.12 |
| rs9929218 | 16 | *G* | 1.1 |
| rs4939827 | 18 | *T* | 1.2 |
| rs10411210 | 19 | *C* | 1.15 |
| rs2423279 | 20 | C | 1.07 |
| rs961253 | 20 | *A* | 1.12 |
| rs4813802 | 20 | *G* | 1.09 |
| rs4925386 | 20 | *C* | 1.08 |
| rs5934683 | X | *T* | 1.07 |

**Table 4. Prostate Cancer**

| SNP | CHR | Risk Allele | OR/Risk |
|---|---|---|---|
| rs1218582 | 1 | G | 1.06 |
| rs4245739 | 1 | A | 1.1 |
| rs10187424 | 2 | A | 1.19 |
| rs721048 | 2 | A | 1.15 |
| rs1465618 | 2 | A | 1.27 |
| rs11902236 | 2 | A | 1.07 |
| rs12621278 | 2 | A | 1.35 |
| rs3771570 | 2 | A | 1.12 |
| rs2292884 | 2 | G | 1.14 |
| rs2660753 | 3 | T | 1.18 |
| rs7611694 | 3 | A | 1.1 |
| rs10934853 | 3 | A | 1.12 |
| rs6763931 | 3 | T | 1.18 |
| rs10936632 | 3 | A | 1.14 |
| rs1894292 | 4 | G | 1.1 |
| rs17021918 | 4 | C | 1.19 |
| rs7679673 | 4 | C | 1.19 |
| rs2121875 | 5 | G | 1.09 |
| rs6869841 | 5 | A | 1.07 |
| rs130067 | 6 | G | 1.2 |
| rs3096702 | 6 | A | 1.07 |
| rs2273669 | 6 | G | 1.07 |
| rs9364554 | 6 | T | 1.17 |
| rs1933488 | 6 | A | 1.12 |
| rs10486567 | 7 | G | 1.19 |
| rs12155172 | 7 | A | 1.11 |
| rs6465657 | 7 | C | 1.12 |
| rs2928679 | 8 | A | 1.53 |
| rs1512268 | 8 | A | 1.23 |
| rs11135910 | 8 | A | 1.11 |
| rs1447295 | 8 | A | 2.23 |
| rs16901979 | 8 | A | 1.79 |
| rs6983267 | 8 | G | 1.58 |
| rs620861 | 8 | C | 1.28 |
| rs10086908 | 8 | T | 1.25 |
| rs16902094 | 8 | G | 1.21 |
| rs1571801 | 9 | T | 1.36 |
| rs10993994 | 10 | T | 1.57 |
| rs3850699 | 10 | A | 1.1 |
| rs4962416 | 10 | C | 1.46 |
| rs7127900 | 11 | A | 1.28 |
| rs10896449 | 11 | G | 1.41 |
| rs12418451 | 11 | A | 1.36 |
| rs11568818 | 11 | A | 1.1 |
| rs1270884 | 12 | A | 1.07 |
| rs10875943 | 12 | C | 1.18 |
| rs902774 | 12 | A | 1.17 |
| rs8008270 | 14 | G | 1.12 |
| rs7141529 | 14 | G | 1.09 |
| rs684232 | 17 | G | 1.1 |
| rs4430796 | 17 | A | 1.22 |
| rs11649743 | 17 | G | 1.5 |
| *rs138213197* | 17 | T | 10 |
| rs11650494 | 17 | A | 1.15 |
| rs1859962 | 17 | G | 1.2 |
| rs7241993 | 18 | G | 1.09 |
| rs2735839 | 19 | G | 1.2 |
| rs8102476 | 19 | C | 1.12 |
| rs887391 | 19 | T | 1.15 |
| rs6062509 | 20 | A | 1.12 |
| rs2427345 | 20 | G | 1.06 |
| rs5759167 | 22 | G | 1.14 |
| rs9623117 | 22 | C | 1.18 |
| rs5945619 | X | C | 1.19 |
| rs2405942 | X | A | 1.14 |
| rs5919432 | X | A | 1.09 |

**Table 5. Glioma**

| SNP | CHR | Risk Allele | OR/Risk |
|---|---|---|---|
| rs2736100 | 5 | C | 1.27 |
| rs4295627 | 8 | G | 1.36 |
| rs4977756 | 9 | G | 1.24 |
| rs498872 | 11 | T | 1.18 |
| rs6010620 | 20 | G | 1.28 |
| rs4809324 | 20 | C | 1.27 |

**Table 6. Neuroblastoma**

| SNP | CHR | Risk Allele | OR/Risk |
|---|---|---|---|
| rs6435862 | 2 | G | 1.68 |
| rs6939340 | 6 | G | 1.37 |
| rs4336470 | 6 | C | 1.26 |
| rs17065417 | 6 | A | 1.38 |
| rs110419 | 11 | A | 1.34 |

**Table 7. Chronic Lymphocytic Leukemia**

| SNP | CHR | Risk Allele | OR/Risk |
|---|---|---|---|
| rs17483466 | 2 | G | 1.39 |
| rs13397985 | 2 | G | 1.41 |
| rs757978 | 2 | A | 1.39 |
| rs872071 | 6 | G | 1.54 |
| rs210142 | 6 | C | 1.4 |
| rs2456449 | 8 | G | 1.28 |
| rs735665 | 11 | A | 1.45 |
| rs7176508 | 15 | A | 1.37 |
| rs391525 | 16 | A | 1.82 |
| rs11083846 | 19 | A | 1.35 |

**Table 8. Pancreatic Cancer**

| SNP | CHR | Risk Allele | OR/Risk |
|---|---|---|---|
| rs3790844 | 1 | A | 1.3 |
| rs505922 | 9 | C | 1.2 |
| rs9543325 | 13 | C | 1.26 |

**Table 9. Non-Hodgkin Lymphoma**

| SNP | CHR | Risk Allele | OR/Risk |
|---|---|---|---|
| rs2647012 | 6 | C | 2 |
| rs6457327 | 6 | C | 1.7 |
| rs10484561 | 6 | G | 1.12 |

**Table 10. Bladder Cancer**

| SNP | CHR | Risk Allele | OR/Risk |
|---|---|---|---|
| rs3752749 | 4p16 | T | 1.2 |
| rs9642880 | 8q24 | T | 1.21 |
| rs2294008 | 8q24 | T | 1.13 |
| rs710521 | 3q28 | T | 1.14 |
| rs1495741 | 8p22 | A | 1.15 |
| rs17674580 | 18q12 | T | 1.17 |
| rs8102137 | 19q12 | C | 1.13 |
| rs1014971 | 22q13 | T | 1.14 |

**Table 11. Renal Cell Carcinoma**

| SNP | CHR | Risk Allele | OR/Risk |
|---|---|---|---|
| rs7579899 | 2 | A | 1.15 |
| rs12105918 | 2 | C | 1.29 |
| rs7105934 | 11 | G | 1.44 |
| rs718314 | 12 | G | 1.19 |
| rs4765623 | 12 | T | 1.15 |

**Table 12. Ovarian Cancer**

| SNP | CHR | Risk Allele | OR/Risk |
|---|---|---|---|
| rs2072590 | 2 | T | 1.16 |
| rs11782652 | 8 | G | 1.19 |
| rs10088218 | 8 | G | 1.19 |
| rs3814113 | 9 | T | 1.22 |
| rs1243180 | 10 | A | 1.1 |
| rs757210 | 17 | A | 1.05 |
| rs8170 | 19 | T | 1.18 |

**Table 13. Melanoma**

| SNP | CHR | Risk Allele | OR/Risk |
|---|---|---|---|
| rs7412746 | 1 | T | 1.12 |
| rs13016963 | 2 | A | 1.14 |
| rs1801516 | 11 | G | 1.19 |
| rs1393350 | 11 | A | 1.29 |
| rs1129038 | 15 | A | 1.45 |
| rs16953002 | 16 | A | 1.16 |
| rs258322 | 16 | A | 1.67 |
| rs4785751 | 16 | G | 1.43 |
| rs910873 | 20 | T | 1.75 |
| rs45430 | 21 | A | 1.14 |
| rs2284063 | 22 | A | 1.2 |

**Table 14. Hodgkin Lymphoma**

| SNP | CHR | Risk Allele | OR/Risk |
|---|---|---|---|
| c | 2 | G | 1.22 |
| rs6903608 | 6 | G | 1.7 |
| rs2248462 | 6 | G | 1.64 |
| rs9268542 | 6 | G | 1.6 |
| rs204999 | 6 | A | 2 |
| rs2019960 | 8 | G | 1.33 |
| rs2395185 | 6 | G | 1.82 |

**Table 15. Acute Lymphocytic Leukemia**

| SNP | CHR | Risk Allele | OR/Risk |
|---|---|---|---|
| rs4132601 | 7 | C | 1.69 |
| rs7088318 | 10 | A | 1.4 |
| rs7089424 | 10 | C | 1.65 |

**Table 16. Thyroid Cancer**

| SNP | CHR | Risk Allele | OR/Risk |
|---|---|---|---|
| rs966423 | 8 | C | 1.34 |
| rs2439302 | 8 | G | 1.36 |
| rs965513 | 9 | A | 1.75 |
| rs944289 | 14 | T | 1.37 |
| rs116909374 | 14 | T | 2.03 |

**Table 17. Testicular Germ-Cell Cancer**

| SNP | CHR | Risk Allele | OR/Risk |
|---|---|---|---|
| rs2072499 | 1 | G | 1.19 |
| rs3790672 | 2 | C | 1.2 |
| rs10510452 | 3 | A | 1.24 |
| rs17021463 | 4 | T | 1.19 |
| rs2720460 | 4 | A | 1.24 |
| rs2736100 | 5 | T | 1.33 |
| rs4635969 | 5 | T | 1.54 |
| rs3805663 | 5 | T | 1.25 |
| rs4624820 | 5 | A | 1.37 |
| rs210138 | 6 | G | 1.5 |
| rs12699477 | 7 | C | 1.21 |
| rs7010162 | 8 | G | 1.22 |
| rs7040024 | 9 | A | 1.7 |
| rs2900333 | 12 | C | 1.27 |
| rs995030 | 12 | G | 2.55 |
| rs8046148 | 16 | G | 1.32 |
| rs4888262 | 16 | C | 1.26 |
| rs9905704 | 17 | T | 1.27 |
| rs2839186 | 21 | T | 1.26 |

**Table 18.**

| **Comparison of family history (FH) and genetic risk score (GRS) in measuring risk for cancer** | | | | | | | |
|---|---|---|---|---|---|---|---|
| | **FH** | | | | **GRS** | | |
| **Cancer site** | **Prevalence (%)** | **RR** | **GV** | **AUC** | **No. of SNPs** | **GV** | **AUC** |
| Breast Cancer | 11.80 | 1.44 | 0.008 | 0.526 | 67 | 0.143 | 0.605 |
| Lung Cancer | 10.10 | 1.70 | 0.013 | 0.532 | 4 | 0.099 | 0.588 |
| Colorectal Cancer | 9.40 | 1.72 | 0.013 | 0.532 | 26 | 0.06 | 0.569 |
| Prostate Cancer | 7.30 | 1.72 | 0.008 | 0.525 | 66 | 0.439 | 0.68 |
| Glioma | 6.10 | 1.60 | 0.008 | 0.526 | 6 | 0.077 | 0.578 |
| Neuroblastoma | 5.00 | 9.20 | 0.156 | 0.61 | 5 | 0.163 | 0.612 |
| Chronic lymphoblastic leukemia | 5.00 | 7.52 | 0.129 | 0.6 | 10 | 0.358 | 0.664 |
| Pancreatic Cancer | 2.00 | 1.62 | 0.002 | 0.513 | 3 | 0.027 | 0.546 |
| Non-Hodgkin Lymphoma | 2.00 | 1.50 | 0.002 | 0.513 | 3 | 0.242 | 0.636 |
| Bladder Cancer | 1.70 | 1.32 | 0.001 | 0.507 | 8 | 0.039 | 0.556 |
| Renal Cell Cancer | 1.40 | 2.17 | 0.006 | 0.521 | 5 | 0.032 | 0.55 |
| Ovarian Cancer | 1.10 | 3.10 | 0.007 | 0.524 | 7 | 0.03 | 0.548 |
| Melanoma | 1.00 | 1.74 | 0.001 | 0.509 | 11 | 0.096 | 0.587 |
| Hodgkin Lymphoma | 1.00 | 2.40 | 0.002 | 0.512 | 7 | 0.474 | 0.686 |
| Acute lymphoblastic leukemia | 0.80 | 2.21 | 0.003 | 0.516 | 3 | 0.185 | 0.62 |
| Thyroid Cancer | 0.50 | 2.80 | 0.004 | 0.517 | 5 | 0.121 | 0.597 |
| Testicular Cancer | 0.40 | 3.10 | 0.001 | 0.51 | 19 | 0.203 | 0.625 |

Summary Paragraphs:
1. A method of producing a personalized cancer risk report for a subject, comprising:
   a) determining, from a nucleic acid sample obtained from the subject, a genotype for the subject at a plurality of biallelic polymorphic loci, wherein each locus of said plurality has an associated allele and an unassociated allele, wherein the genotype is selected from the group consisting of homozygous for the associated allele, heterozygous, and homozygous for the unassociated allele and wherein said plurality of biallelic polymorphic loci is a multiplicity, in any combination, of the single nucleotide polymorphisms in Table 1 (breast), Table 2 (lung), Table 3 (colorectal), Table 4 (prostate), Table 5 (glioma), Table 6 (neuroblastoma), Table 7 (chronic lymphocytic leukemia), Table 8 (pancreatic), Table 9 (non-Hodgkin lymphoma), Table 10 (bladder), Table 11 (renal) Table 12 (ovarian), Table 13 (melanoma), Table 14 (Hodgkin lymphoma), Table 15 (acute lymphocytic leukemia), Table 16 (thyroid), and/or Table 17 (testicular);
   b) calculating a genetic risk score (GRS) for the subject based on the genotype determined for said plurality of biallelic polymorphic loci of step (a); and
   c) producing a personalized cancer risk report for the subject based on the GRS calculated in step (b).
2. A method of identifying a subject as having an increased risk of developing breast cancer, lung cancer, colorectal cancer, prostate cancer, glioma, neuroblastoma, chronic lymphocytic leukemia, pancreatic cancer, non-Hodgkin lymphoma, bladder cancer, renal cancer, ovarian cancer, melanoma, Hodgkin lymphoma, acute lymphocytic leukemia, thyroid cancer and/or testicular cancer, comprising:
   a) determining, from a nucleic acid sample obtained from the subject, a genotype for the subject at a plurality of biallelic polymorphic loci, wherein each locus of said plurality has an associated allele and an unassociated allele, wherein the genotype is selected from the group consisting of homozygous for the associated allele, heterozygous, and homozygous for the unassociated allele and wherein said plurality of biallelic polymorphic loci is a multiplicity, in any combination, of the single nucleotide polymorphisms in Table 1 (breast), Table 2 (lung), Table 3 (colorectal), Table 4 (prostate), Table 5 (glioma), Table 6 (neuroblastoma), Table 7 (chronic lymphocytic leukemia), Table 8 (pancreatic), Table 9 (non-Hodgkin lymphoma), Table 10 (bladder), Table 11 (renal) Table 12 (ovarian), Table 13 (melanoma), Table 14 (Hodgkin lymphoma), Table 15 (acute lymphocytic leukemia), Table 16 (thyroid), Table 17 (testicular); and
   b) calculating a genetic risk score (GRS) for the subject based on the genotype determined for each plurality of biallelic polymorphic loci in step (a), wherein a GRS of greater than 1.0 identifies the subject as having an increased risk of developing the type of cancer associated with said GRS of greater than 1.0, thereby identifying the subject as having an increased risk of developing breast cancer, lung cancer, colorectal cancer, prostate cancer, glioma, neuroblastoma, chronic lymphocytic leukemia, pancreatic cancer, non-Hodgkin lymphoma, bladder cancer, renal cancer, ovarian cancer, melanoma, Hodgkin lymphoma, acute lymphocytic leukemia, thyroid cancer and/or testicular cancer.
3. The method of paragraph 1 or paragraph 2, wherein the determining step comprises receiving genotype data from genotyping apparatus.
4. The method of any of paragraphs 1-2, wherein the plurality of biallelic polymorphic loci includes every single nucleotide polymorphism of Tables 1 through 17.
5. The method of any of paragraphs 1-2, wherein the subject has a family history of breast cancer, lung cancer, colorectal cancer, prostate cancer, glioma, neuroblastoma, chronic lymphocytic leukemia, pancreatic cancer, non-Hodgkin lymphoma, bladder cancer, renal cancer, ovarian cancer, melanoma, Hodgkin lymphoma, acute lymphocytic leukemia, thyroid cancer and/or testicular cancer.
6. The method of any of paragraphs 1-2, wherein the subject is not previously considered or identified to be at high risk of having or developing breast cancer, lung cancer, colorectal cancer, prostate cancer, glioma, neuroblastoma, chronic lymphocytic leukemia, pancreatic cancer, non-Hodgkin lymphoma, bladder cancer, renal cancer, ovarian cancer, melanoma, Hodgkin lymphoma, acute lymphocytic leukemia, thyroid cancer and/or testicular cancer.
7. The method of paragraph 6, further comprising the step of screening the subject for the cancer(s) associated with said GRS of greater than 1.0 according to a protocol recommended for a subject considered or identified to be at high risk of having or developing the cancer(s) associated with said GRS of greater than 1.0.
8. The method of any of paragraphs 2-7, further comprising the step of administering a prophylactic treatment to the subject that is specific for the cancer(s) associated with said GRS of greater than 1.0.
9. A kit comprising reagents and instructions for carrying out the method of any preceding paragraph.
10. A computer program product comprising:
   a computer readable storage medium having computer readable code embodied in the medium, the computer code comprising:
   computer readable code to perform operations to carry out the method of any of paragraphs 1-8.
11. A computer system, comprising:
   a processor; and
   a memory coupled to the processor, the memory comprising computer readable program code embodied therein that, when executed by the processor, causes the processor to perform operations to carry out the method of any of paragraphs 1-8.

## Claims

1. A method of producing a personalized cancer risk report for a subject, comprising:
a) determining, from a nucleic acid sample obtained from the subject, a genotype for the subject at a plurality of biallelic polymorphic loci, wherein each locus of said plurality has an associated allele and an unassociated allele, wherein the genotype is selected from the group consisting of homozygous for the associated allele, heterozygous, and homozygous for the unassociated allele and wherein said plurality of biallelic polymorphic loci is a multiplicity, in any combination, of the single nucleotide polymorphisms in Table 3 (colorectal);
b) calculating a genetic risk score (GRS) for the subject based on the genotype determined for said plurality of biallelic polymorphic loci of step (a); and
c) producing a personalized cancer risk report for the subject based on the GRS calculated in step (b).

2. A method of identifying a subject as having an increased risk of developing colorectal cancer, comprising:
a) determining, from a nucleic acid sample obtained from the subject, a genotype for the subject at a plurality of biallelic polymorphic loci, wherein each locus of said plurality has an associated allele and an unassociated allele, wherein the genotype is selected from the group consisting of homozygous for the associated allele, heterozygous, and homozygous for the unassociated allele and wherein said plurality of biallelic polymorphic loci is a multiplicity, in any combination, of the single nucleotide polymorphisms in Table 3 (colorectal), ; and
b) calculating a genetic risk score (GRS) for the subject based on the genotype determined for each plurality of biallelic polymorphic loci in step (a), wherein a GRS of greater than 1.0 identifies the subject as having an increased risk of developing the type of cancer associated with said GRS of greater than 1.0, thereby identifying the subject as having an increased risk of developing colorectal cancer, .

3. The method of claim 1 or claim 2, wherein the determining step comprises receiving genotype data from genotyping apparatus.

4. The method of claim 1 or claim 2, wherein the plurality of biallelic polymorphic loci includes at least 4 single nucleotide polymorphism of Table 3.

5. The method of any one of claims 1 -2, wherein the plurality of biallelic polymorphic loci includes every single nucleotide polymorphism of Table 3.

6. The method of any one of claims 1-2, wherein the subject has a family history of colorectal cancer, .

7. The method of any one of claims 1-2, wherein the subject is not previously considered or identified to be at high risk of having or developing colorectal cancer, .

8. The method of claim 7, further comprising the step of screening the subject for the cancer(s) associated with said GRS of greater than 1.0 according to a protocol recommended for a subject considered or identified to be at high risk of having or developing the cancer(s) associated with said GRS of greater than 1.0.

9. Postmenopausal female hormone supplements for use in a method of prophylactic treatment for colorectal cancer in a subject with a GRS of greater than 1.0 identified by the method of any one of claims 2-8.

10. Nonsteroidal anti-inflammatory drugs for use in a method of prophylactic treatment for colorectal cancer in a subject with a GRS of greater than 1.0 identified by the method of any one of claims 2-8.

11. A kit comprising reagents and instructions for carrying out the method of any one of claims 1-8.

12. A computer program product for producing a personalized cancer risk report, comprising:
a computer readable storage medium having computer readable code embodied in the medium, the computer code comprising:
computer readable code to perform operations to carry out the method of any one of claims 1-8.

13. A computer system for producing a personalized cancer risk report, comprising:
a processor; and
a memory coupled to the processor, the memory comprising computer readable program code embodied therein that, when executed by the processor, causes the processor to perform operations to carry out the method of any of claims 1-8.
